(19) Europäisches Patentamt
European Patent Office
Office européen des brevets

(11) Veröffentlichungsnummer: **0 051 202**
**B1**

(12) **EUROPÄISCHE PATENTSCHRIFT**

(45) Veröffentlichungstag der Patentschrift: **09.11.83**

(51) Int. Cl.³: **C 07 C 147/08**, C 07 C 147/04

(21) Anmeldenummer: **81108529.9**

(22) Anmeldetag: **20.10.81**

(54) **Verfahren zur Herstellung von 2,3-Dichlor-sulfonyl-acrylnitrilen.**

(30) Priorität: **31.10.80 DE 3041155**

(43) Veröffentlichungstag der Anmeldung: **12.05.82 Patentblatt 82/19**

(45) Bekanntmachung des Hinweises auf die Patenterteilung: **09.11.83 Patentblatt 83/45**

(84) Benannte Vertragsstaaten: **CH DE FR GB LI**

(56) Entgegenhaltungen:
**EP - A - 0 001 312**
**DE - A - 2 500 265**
**US - A - 3 078 298**

(73) Patentinhaber: **BAYER AG, Zentralbereich Patente, Marken und Lizenzen, D-5090 Leverkusen 1, Bayerwerk (DE)**

(72) Erfinder: **Oeckl, Siegfried, Dr., Andreas-Gryphius-Strasse 9, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 051 202 B1

BUNDESDRUCKEREI BERLIN

Verfahren zur Herstellung von 2,3-Dichlor-sulfonylacrylnitrilen

Die Erfindung betrifft ein Verfahren zur Herstellung von 2,3-Dichlor-sulfonyl-acrylnitrilen durch Chlorierung der entsprechenden 3-Sulfonyl-propionitrile.

Es ist aus der EEP 0 001 312 bekannt, 2,3-Dichlor-3-phenyl-sulfonyl-acrylnitrile durch Umsetzung von Thiolen mit Trichloracrylnitril und nachfolgender Oxidation zu den entsprechenden Sulfonen herzustellen. Das dort beschriebene Verfahren umfaßt nicht die Herstellung des unsubstituierten 3-Phenyl-sulfonyl-acrylnitrils.

Bei der Herstellung von substituierten 3-Phenyl-sulfonyl-acrylnitrilen nach der EEP 0 001 312 sind mehrere aufwendige Reaktionsstufen erforderlich.

Es wurde dagegen ein einfaches Verfahren zur Herstellung von 2,3-Dichlor-sulfonyl-acrylnitrilen gefunden, das dadurch gekennzeichnet ist, daß man 3-Sulfonylpropionitrile der Formel

$$R^1-SO_2-CH_2-CH_2-CN$$

worin

$R^1$ gegebenenfalls substituiertes Aryl, Aralkyl, Alkyl oder Cycloalkyl bedeutet,

in Gegenwart eines basischen Katalysators mit einem Chlorierungsmittel bei höherer Temperatur umsetzt.

Als Alkyl seien geradkettige oder verzweigte Kohlenwasserstoffreste mit 1 bis 12 Kohlenstoffatomen, bevorzugt Niederalkylreste (1 bis etwa 6 Kohlenstoffatome) genannt. Alkylreste sind beispielsweise Methyl, Ethyl, Propyl, Isopropyl, Butyl, Isobutyl, Pentyl, Isopentyl, Hexyl, Isohexyl, Octyl, Isooctyl und Undecyl. Insbesondere bevorzugte Alkylreste sind Methyl und Ethyl.

Als Aryl seien aromatische Reste mit 6 bis 18 Kohlenstoffatomen genannt, die gegebenenfalls linear miteinander verknüpft oder kondensiert sein können. Beispielsweise seien Phenyl, Biphenyl und Naphthyl genannt. Insbesondere wird der Phenylrest bevorzugt.

Als Aralkyl seien Reste mit 7 bis 14, bevorzugt 7 bis 10, Kohlenstoffatomen genannt, wobei der aromatische Teil des Aralkyls 5 bis 10, bevorzugt 5 und 6, Kohlenstoffatome und der aliphatische Teil 1 bis 6, bevorzugt 1 bis 3, Kohlenstoffatome enthalten kann. Beispielsweise seien Benzyl, Ethylphenyl und $\alpha$- und $\beta$-Methyl-naphthyl genannt. Insbesondere wird der Benzylrest bevorzugt.

Als Cycloalkylreste seien cyclische aliphatische Kohlenwasserstoffreste mit 5 bis 17 Kohlenstoffatomen, bevorzugt 5, 6 und 10 Kohlenstoffatome, genannt. Es ist auch möglich, daß mehrere Cycloalkylreste linear miteinander verknüpft oder kondensiert sein können. Beispielsweise seien Cyclopentyl, Cyclohexyl und der Decalinrest genannt. Insbesondere bevorzugter Cycloalkylrest ist der Cyclohexylrest.

3-Sulfonyl-propionitrile sind aus Roczniki Chemii 30, 243 (1956) bekannt. Sie können beispielsweise durch Umsetzung von Sulfinsäuren oder Sulfinaten mit Acrylnitril in wäßrigem Medium erhalten werden.

Bevorzugte 3-Sulfonyl-propionitrile sind Verbindungen der Formel

$$R^2-SO_2-CH_2-CH_2-CN$$

worin

$R^2$ gegebenenfalls substituiertes Phenyl, Naphthyl, Benzyl, Naphthylmethyl, geradkettiges oder verzweigtes Niederalkyl oder Cycloalkyl vedeutet.

Insbesondere bevorzugt werden 3-Sulfonyl-propionitrile der Formel

$$R^3-SO_2-CH_2-CH_2-CN$$

worin

$R^3$ Phenyl, 4-Chlor-phenyl, 2,5-Dichlor-phenyl, Benzyl, Methyl, Ethyl und Cyclohexyl bedeutet.

Beispielsweise seien die folgenden 3-Sulfonyl-propionitrile genannt:

3-Phenyl-sulfonyl-propionitril,
3-Methyl-sulfonyl-propionitril,
(2,5-Dichlor-phenyl)-sulfonyl-propionitril,
(3-Nitro-phenyl)-sulfonyl-propionitril.

Basische Katalysatoren für das erfindungsgemäße Verfahren sind bevorzugt Pyridin-Basen wie Pyridin, Picolin und Chinolin.

Insbesondere wird Pyridin als basischer Katalysator bevorzugt.

Die Menge des basischen Katalysators im Reaktionsgemisch kann in weiten Grenzen schwanken. Im allgemeinen verwendet man 0,1 bis 20 Gew.-%, bevorzugt 1 bis 10 Gew.-% an basischem Katalysator, bezogen auf das 3-Sulfonyl-propionitril.

Als Chlorierungsmittel für das erfindungsgemäße Verfahren seien die üblichen Chlorierungsmittel, wie elementares Chlor und die Phosphorchloride, genannt. Bevorzugte Chlorierungsmittel für das erfindungsgemäße Verfahren sind die Phosphorchloride, insbesondere Phosphortrichlorid und Phosphorpentachlorid, wobei es zweckmäßig sein kann nur einen Teil der benötigten Menge Phosphorchlorid einzusetzen und durch Einleiten von Chlor das bereits umgesetzte Phosphorpentachlorid wieder zu chlorieren.

Besonders bevorzugt für das erfindungsgemäße Verfahren ist eine Chlorierung der 3-Sulfonyl-propionitrile mit Chlor und/oder Phosphorchloriden in Gegenwart von Pyridin.

Das Gewichtsverhältnis von Chlorierungsmittel zu basischem Katalysator kann im Bereich von 10 : 1 bis 1 : 10 schwanken. Bevorzugt wird jedoch die 1- bis 10fache Menge des Chlorierungsmittels, bezogen auf das Pyridin, eingesetzt.

Im allgemeinen wird der Katalysator zu Beginn der Chlorierung zugesetzt. In manchen Fällen kann es zweckmäßig sein, im Laufe der Chlorierung zusätzlich noch basischen Katalysator zuzusetzen.

Für das erfindungsgemäße Verfahren setzt man im allgemeinen mindestens 3 Mol der Chlorierungsmittel ein. Es kann zweckmäßig sein, die Chlorierungsmittel im Überschuß einzusetzen, wenn beispielsweise der entstehende Chlorwasserstoff einen Teil der Chlorierungsmittel mitreißt. Zweckmäßierweise verwendet man für das erfindungsgemäße Verfahren 3 bis 10, bevorzugt 4 bis 5 Mol der Chlorierungsmittel, bezogen auf das 3-Sulfonyl-propionitril.

Die Chlorierngsmittel werden bevorzugt so dosiert, daß sie sofort mit dem zu chlorierenden Produkt reagieren. Ein Überschuß an Chlorierungsmittel im Reaktionsmedium wirkt im allgemeinen beschleunigend auf die Reaktion.

Das erfindungsgemäße Verfahren wird bei höheren Temperaturen durchgeführt. Es ist zweckmäßig die Temperatur so zu wählen, daß eine genügend rasche Chlorierung erfolgt, ohne daß schon Neben- und Zersetzungsreaktionen eine störende Rolle spielen. Bevorzugt führt man das erfindungsgemäße Verfahren im Temperaturbereich von 30 bis 130° C, insbesondere bevorzugt im Temperaturbereich von 80 bis 120° C, durch.

Das erfindungsgemäße Verfahren wird üblicherweise bei Normaldruck durchgeführt. Es ist jedoch auch möglich, die Umsetzung bei einem Unterdruck (beispielsweise bis zu 0,5 bar) oder einem Überdruck (beispielsweise bis zu 5 bar) durchzuführen.

Bei der erfindungsgemäßen Herstellung der 2,3-Dichlorsulfonyl-acrylnitrile aus den 3-Sulfonyl-propionitrilen können als Zwischenprodukt 2,2-Dichlor-sulfonyl-propionitrile der Formel

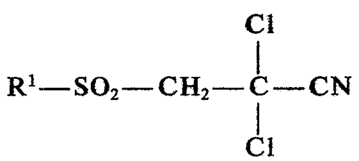

worin

$R^1$ die obengenannte Bedeutung hat,

auftreten. Diese Zwischenprodukte werden im allgemeinen nicht isoliert und reagieren sofort weiter zu den 2,3-Dichlor-sulfonyl-acrylnitrilen. Selbstverständlich ist es jedoch im Rahmen des erfindungsgemäßen Verfahrens auch möglich, anstelle der 3-Sulfonyl-propionitrile die 2,2-Dichlor-sulfonyl-propionitrile einzusetzen. Die 2,2-Dichlor-sulfonyl-propionitrile sind beispielsweise aus der DE-OS 2 500 265 bekannt und können beispielsweise durch Chlorierung von 2-Chlor-sulfonylpropionitrilen hergestellt werden.

Das erfindungsgemäße Verfahren kann beispielsweise anhand der folgenden Reaktionsgleichung erläutert werden:

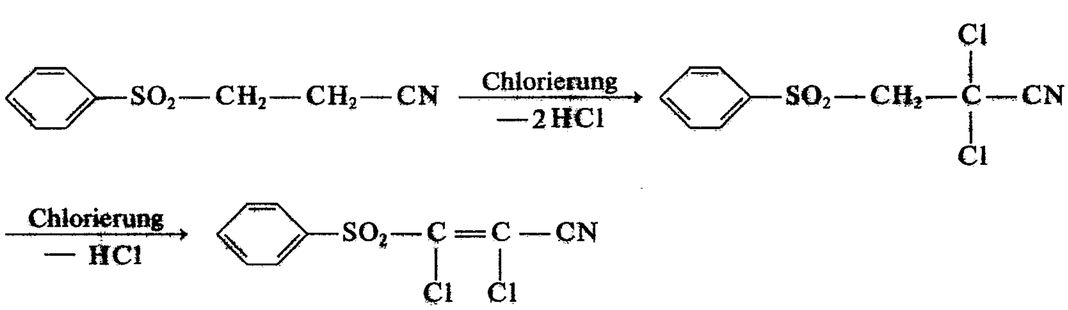


Das erfindungsgemäße Verfahren kann beispielsweise wie folgt durchgeführt werden:

Die Sulfonyl-propionitrile werden bis zur Reaktionstemperatur erwärmt, vorzugsweise bis zu der Temperatur bei der sie geschmolzen vorliegen. Dann gibt man den basischen Katalysator zu. Darauf leitet man etwa die Hälfte des Chlorierungsmittels schnell ein, so daß die Umsetzung rasch abläuft. Die weitere Zugabe des Chlorierungsmitttels führt man vorzugsweise bei etwas niedrigerer

Temperatur entsprechend dem Fortschreiten der Umsetzung zu. Nach Beendigung der Umsetzung kühlt man bis auf Raumtemperatur ab und behandelt das Reaktionsgemisch mit Wasser. Nach Abtrennen der wäßrigen Phase erhält man aus der organischen Phase das 2,3-Dichlorsulfonyl-acrylnitril.

Nach dem erfindungsgemäßen Verfahren fällt das 2,3-Dichlor-sulfonyl-acrylnitril mit großer Reinheit an. Selbstverständlich ist es jedoch möglich, zur Erzielung höherer Reinheiten das erfindungsgemäße Produkt nach üblichen Aufbereitungsmethoden, beispielsweise durch Umkristallisation aus Ethanol, weiter zu reinigen.

Es ist überraschend, daß unter den erfindungsgemäßen Bedingungen die Reaktion nicht auf der Stufe der 2,2-Dichlor-sulfonyl-propionitrile stehenbleibt, sondern daß bei der Weiterchlorierung Chlorwasserstoff abgespalten wird und 2,2-Dichlor-sulfonyl-acrylnitrile entstehen.

Nach dem erfindungsgemäßen Verfahren können 2,3-Dichlor-sulfonyl-acrylnitrile der Formel

$$R^1-SO_2-CCl=CCl-CH$$

worin
$R^1$ die obengenannte Bedeutung hat,
entstehen.

Nach dem erfindungsgemäßen Verfahren kann überraschenderweise das neue 2,3-Dichlor-3-phenyl-sulfonyl-acrylnitril hergestellt werden.

Die nach dem erfindungsgemäßen Verfahren hergestellten 2,3-Dichlor-sulfonyl-acrylnitrile haben, wie aus der EEP 0 001 312 bekannt ist, eine mikrobizide Wirkung.

### Beispiel 1

#### 2,3-Dichlor-3-phenylsulfonyl-acrylnitril

In einem 500-ml-Vierhalskolben wurden 195 g (1 Mol) 3-Phenylsulfonylpropionitril bei 100° C geschmolzen und 14 g $PCl_3$ und 4,2 g Pyridin als Katalysator zugesetzt. Dann wurden in 9 Stunden 150 g (2,1 Mol) Chlor bei 100 bis 90° C eingeleitet. Anschließend wurden noch einmal 7 g $PCl_3$ und 2 g Pyridin zugesetzt und bei 80° C in 12 Stunden nochmals 160 g (2,25 Mol) Chlor eingeleitet, wobei ein Teil des Chlors mit dem entweichenden HCl-Gas mitgerissen wurde. Nun wurden bei 20° C unter Kühlung 200 ml Wasser zugetropft. Die Phasen wurden bei 90° C getrennt und der Waschvorgang mit 200 ml Wasser wiederholt. Zur Zerstörung von Benzolsulfochlorid wurde nun 5 Stunden mit 200 ml Wasser gekocht, die organische Phase abgetrennt und im Vakuum getrocknet. Es ergaben sich 227 g (87%) eines dunkelbraunen rohen Öls, das beim Stehen über Nacht erstarrte. Nach Erwärmung und Auflösung in 50 ml Ethanol, Abkühlen mit Eis und Absaugen des Niederschlags wurden 135 g (51%) helle Kristalle, Fp. 72° C, erhalten.

### Beispiel 2

#### 2,3-Dichlor-3-methylsulfonyl-acrylnitril

In einem 100-ml-Vierhalskolben wurden 31 g (0,23 Mol) 3-Methylsulfonylpropionitril bei 100° C geschmolzen und 2,2 g $PCl_3$ und 0,7 g Pyridin zugesetzt. Dann wurden bei 100° C in 7 Stunden 34 g Chlor (0,48 Mol) eingeleitet. Nach erneutem Zusatz von 1,1 g $PCl_3$ und 0,4 g Pyridin wurden nochmals 10 g Chlor nachgeleitet. Nach Abkühlen wurde mit 30 ml Methylenchlorid verdümnt, zweimal mit je 50 ml Wasser ausgeschüttelt und die organische Phase eingeengt. Man erhält 30 g eines dunkelbraunen Öles aus dem sich nach Verrühren mit wenig Ethanol 9,5 g helle Kristalle abscheiden (Fp: 110 bis 114° C).

### Beispiel 3

#### 2,3-Dichlor-3-(2,5-Dichlorphenylsulfonyl)-acrylnitril

In einem 250-ml-Vierhalskolben wurden 60 g (0,23 Mol) 3-(2,5-Dichlorphenylsulfonyl)-propionitril bei 135° C aufgeschmolzen. Dann wurden 3,2 g $PCl_3$ und 1 g Pyridin zugegeben. Anschließend wurden dann bei 130° C 49 g (0,69 Mol) Chlor innerhalb von 17,5 Stunden eingeleitet. Nach DC war nun kaum noch Ausgangsprodukt vorhanden. Nun wurde kurz im Wasserstrahlvakuum entgast, abgekühlt, der Kolbeninhalt mit Methylenchlorid und Wasser ausgeschüttelt, die organische Phase abgetrennt, über $Na_2SO_4$ getrocknet und eingeengt. Es verblieben 65 g (85%) eines dunkelbraunen, viskosen Öls. Nach Säulenchromatographie an Kieselgel wurden daraus 50 g (66%) eines analysenreinen, hellen Öls gewonnen. Bei längerem Stehen bildeten sich Kristalle vom Fp. 84°.

## Patentansprüche

1. Verfahren zur Herstellung von 2,3-Dichlor-sulfonylacrylnitrilen, dadurch gekennzeichnet, daß man 3-Sulfonyl-propionitrile der Formel

$$R^1-SO_2-CH_2-CH_2-CN$$

worin
$R^1$ gegebenenfalls substituiertes Aryl, Aralkyl, Alkyl oder Cycloalkyl bedeutet,
in Gegenwart eines basischen Katalysators mit einem Chlorierungsmittel bei höherer Temperatur umsetzt.

2. Verfahren nach Anspruch 1, dadurch gekennzeichnet, daß man als basischen Katalysator eine Pyridinbase verwendet.

3. Verfahren nach den Ansprüchen 1 bis 2, dadurch gekennzeichnet, daß man als basischen Katalysator Pyridin verwendet.

4. Verfahren nach den Ansprüchen 1 bis 3, dadurch gekennzeichnet, daß man als Chlorierungsmittel ein Phosphorchlorid verwendet.

5. Verfahren nach den Ansprüchen 1 bis 4, dadurch gekennzeichnet, daß man die Umsetzung im Temperaturbereich von 30 bis 130° C durchführt.

## Claims

1. Process for the preparation of 2,3-dichlorosulphonyl-acrylonitriles, characterised in that 3-sulphonyl-propionitriles of the formula

$$R^1-SO_2-CH_2-CH_2-CN$$

wherein
$R^1$ denotes optionally substituted aryl, aralkyl, alkyl or cycloalkyl,
are reacted with a chlorination agent in the presence of a basic catalyst at elevated temperature.

2. Process according to Claim 1, characterised in that a pyridine base is employed as the basic catalyst.

3. Process according to Claims 1 to 2, characterised in that pyridine is employed as the basic catalyst.

4. Process according to Claims 1 to 3, characterised in that a phosphorus chloride is employed as the chlorination agent.

5. Process according to Claims 1 to 4, characterised in that the reaction is carried out in the temperature range from 30 to 130° C.

## Revendications

1. Procédé de production de 2,3-dichlorosulfonylacrylonitriles, caractérisé en ce qu'on fait réagir en présence d'un catalyseur basique avec un agent de chloration, à température élevée, des 3-sulfonylpropionitriles de formule

$$R^1-SO_2-CH_2-CH_2-CN$$

dans laquelle
$R^1$ est un groupe aryle, aralkyle, alkyle ou cycloalkyle éventuellement substitué.

2. Procédé suivant la revendication 1, caractérisé en ce qu'on utilise comme catalyseur basique une base du type de la pyridine.

3. Procédé suivant les revendications 1 et 2, caractérisé en ce qu'on utilise la pyridine comme catalyseur basique.

4. Procédé suivant les revendications 1 à 3, caractérisé en ce qu'on utilise un chlorure de phosphore comme agent de chloration.

5. Procédé suivant les revendications 1 à 4, caractérisé en ce qu'on conduit la réaction dans la plage de température de 30 à 130° C.